**Europäisches Patentamt**

**European Patent Office**

**Office européen des brevets**

(19)

(11) Veröffentlichungsnummer: **0 081 209**

**B1**

(12) # EUROPÄISCHE PATENTSCHRIFT

(45) Veröffentlichungstag der Patentschrift:
24.09.86

(21) Anmeldenummer: **82111174.7**

(22) Anmeldetag: **02.12.82**

(51) Int. Cl.⁴: **A 61 N 1/36,** A 61 B 5/02,
G 06 F 15/42

(54) Anordnung zum Beenden einer Tachykardie.

(30) Priorität: 04.12.81 SE 8107269

(43) Veröffentlichungstag der Anmeldung:
15.06.83 Patentblatt 83/24

(45) Bekanntmachung des Hinweises auf die Patenterteilung:
24.09.86 Patentblatt 86/39

(84) Benannte Vertragsstaaten:
DE FR GB NL SE

(56) Entgegenhaltungen:
EP-A-0 023 134
DE-A-2 528 817
DE-A-2 838 360
DE-A-2 845 323
GB-A-2 083 363
US-A-3 942 534
US-A-4 280 502
US-A-4 285 041

(73) Patentinhaber: **Siemens- Elema AB, Röntgenvägen
2, S-171 95 Solna 1 (SE)**
(84) Benannte Vertragsstaaten: **SE**

(73) Patentinhaber: **Siemens Aktiengesellschaft Berlin
und München, Wittelsbacherplatz 2, D-8000
München 2 (DE)**
(84) Benannte Vertragsstaaten: **DE FR GB NL**

(72) Erfinder: **Hard af Segerstad, Christer, Vasavaegen
51, S-175 32 Järfälla (SE)**
Erfinder: **Vallin, Hans, Wedan, S-137 00
Västerhaninge (SE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Anordnung zum Beenden einer Tachykardie gemäss dem Oberbegriff des Patentanspruches 1. Eine derartige Anordnung ist beispielsweise aus der DE-A- 2 845 323 bekannt. Als Tachykardie bezeichnet man die unnatürlich überhöhte Frequenz des Herzschlages, beispielsweise wenn das Herz mehr als 180-mal pro Minute schlägt. Es ist nun bekannt, dass dieser schnelle Herzrhythmus durch elektrische Stimulierungsimpulse gestoppt werden kann. Voraussetzung dafür ist jedoch, dass ein Stimulierungsimpuls innerhalb eines kritischen Zeitintervalles nach einem Herzschlag, einem sogenannten Fenster, an das Herz abgegeben wird. Dieses Fenster kann direkt nach der auf einen Herzschlag folgenden Refraktärzeit des Herzens oder auch erst unmittelbar vor einem nächsten Herzschlag liegen. Aus Untersuchungen ist weiter bekannt, dass die Breite dieses Fensters und deren Lage mit der Körperlage, physischen Aktivitäten, Drogen und ähnlichem variiert. Dabei können für unterschiedliche Bedingungen die Fenster derart liegen, dass Sie sich nicht überlappen. Es ist daher nicht möglich, für jeden Patienten eine feste Stimulierungszeit synchron zum Herzschlag einzustellen.

Bei dem aus der genannten Offenlegungsschrift bekannten Verfahren zum Beenden einer Tachykardie wird die Pause zwischen zwei Stimulierungsimpulsen zunächst so verkürzt, dass die Stimulierungsimpulse etwa mit gleicher Frequenz wie die Tachykardie-Herzschläge erfolgen. Anschliessend wird dann die Pause zwischen den Stimulierungsimpulsen in äquidistanten Schritten von 10 Millisekunden auf das Ausgangsintervall, das einem natürlichen Herzschlag entspricht, zurückgeführt. Damit wird das gesamte Intervall zwischen zwei Tachykardie-Herzschlägen abgerastert. Da die Fenster oft sehr schmal sind, muss bei diesem Verfahren der Abstand zwischen den einzelnen Stimulierungsimpulsen entsprechend klein gewählt werden. Das bedeutet aber, dass zum Ueberstreichen des gesamten möglichen Intervalles eine grosse Anzahl unterschiedliche Impulse notwendig ist.

Aus der US-PS 3 942 534 ist eine ähnliche Anordnung bekannt, mit der das Zeitintervall zwischen der Beendigung der Refraktärzeit und dem nächstfolgenden Herzschlag in äquidistanten Schritten abgerastert wird. Das bedeutet, dass beispielsweise der erste Stimulierungsimpuls mit dem Ende der Refraktärzeit zusammenfällt, der nächste Stimulierungsimpuls 10 Millisekunden später erfolgt, der nächste wiederum 10 Millisekunden später u.s.w. Weiterhin ist aus dieser Schrift bekannt, das die Verzögerungszeit zwischen einem Herzsignal und dem nächstfolgenden Stimulierungsimpuls von einem maximalen Wert in äquidistanten Schritten abgesenkt werden kann, d.h. also dass das gesamte Intervall rückwärts abgerastert wird. Versuche mit nach diesem Prinzip arbeitenden Anordnungen haben gezeigt, dass die Zeit, bis eine Tachykardie beendet wird, in ungünstligen Fällen über 15 Minuten dauern kann.

Weitere Versuche haben gezeigt, dass Tachykardien oft leichter zu stoppen sind, je zeitiger der richtige Stimulierungszeitpunkt getroffen wird. Insbesondere gilt das für Tachykardien, die den AV-Knoten befallen.

Der vorliegenden Erfindung liegt die Aufgabe zugrunde, eine Anordnung der eingangs genannten Art derartzu verbessern, dass das effektiv wirksame Fenster zum Beenden einer Tachykardie wesentlich schneller gefunden werden kant. Diese Aufgabe wird erfindungsgemäss durch die im kennzeichnenden Teil des Anspruches 1 angegebenen Merkmale gelöst. Die Erfindung nutzt dabei die Erkenntnis aus, das das kritische Fenster um so breiter ist, je später es im Intervall zwischen Refraktärzeit und und nachfolgendem Herzschlag liegt. Zunächst wird bei einer Untersuchung die Refraktärzeit des Herzens während einer Tachykardie in bekannter Weise bestimmt und dieser Wert als untere Grenze für die Verzögerung der Stimulierungsimpulse festgesetzt. Die Anordnung selbst bestimmt dann die Differenz zwischen der RR-Zeit bei Tachykardie und dem festgesetzten unteren Grenzwert und teilt das so erhaltene Intervall in eine passende Anzahl Stufen ein (beispielsweise 6 bis 10 Stufen). Im einfachsten Fall können die Stufen durch jeweiliges Halbieren des Restintervalles erhalten werden. Es ist aber auch jede andere geometrische Aufteilung möglich, beispielsweise jeweils Drittel oder anderes. Wesentlich ist nur, dass die Stufen gegen die Refraktärzeit hin immer kürzer werden. Unabhängig, ob die Stimulierungsimpulse von der unteren Verzögerungszeitgrenze beginnen oder von der oberen in jedem Fall wird das gesamte Intervall mit wenigen Schritten überdeckt. Eine besondere Abtastung des Intervalles sieht vor, dass man mit einem mittleren Stimulierungsimpuls beginnt und dann abwechselnd nach links oder rechts mit dabei kleiner respektive grösser werdenden Stufen arbeitet. Diese Art des Abtastens bezeichnen wir als "zentrifugierende" Abtastung.

In Weiterbildung der Erfindung ist vorgesehen, dass die Anordnung einen Speicher zur Aufnahme der unterschiedlichen Verzögerungszeiten aufweist. Ähnlich wie in einer Tabelle können darin die entsprechenden Verzögerungszeiten gelagert werden und nach einem wählbaren Muster zur Verzögerung der aufeinanderfolgenden Stimulierungsimpulse herangezogen werden.

Eine weitere Herabsetzung der Absuchzeit erhält man in Weiterbildung der Erfindung dadurch, dass die zum Beenden einer Tachykardie führende Verzögerungszeit ebenfalls in den Speicher eingebbar ist und dass beim erneuten Auftreten einer Tachykardie der neue Versuch zum Beenden derselben bei dieser Einstellung begonnen wird. Insbesondere hierbei ist es vorteilhaft, wenn die weiteren Verzögerungszeiten zentrifugal durchlaufen werden.

Mit grosser Wahrscheinlichkeit liegt das kritische Fenster für die erneute Tachykardie, wenn nicht an gleicher Stelle, so doch zumindest in der Nähe derselben. Da der gespeicherte wert zum Beenden der früheren Tachykardie nicht unbedingt der mittlere aller möglichen Verzögerungszeitwerte ist, existiert nach beiden Richtungen von diesem Wert eine unterschiedliche Anzahl möglicher Stufen. Beim zentrifugalen Durchlaufen aller möglichen Verzögerungszeiten stösst man in einer Richtung an ein Grenze, während in der anderen Richtung noch Stufen frei sind. In einem solchen Fall kann beispielsweise die Anordnung den erreichten Grenzwert mehrfach wiederholen, bis auch die Stufen auf der anderen Seite abgefragt sind oder direkt nur auf der verbleibenden Seite in einer Richtung weitergehen.

In einer vorteilhaften Weiterbildung der Erfindung ist vorgesehen, dass synchron zu den Herzsignalen nicht nur jeweils ein, sondern mehrere Stimulierungsimpulse erzeugt werden, wobei der Abstand dieser Impulse untereinander fest vorgebbar ist. Sollte beispielsweise nach dem Durchlaufen der verschiedenen Verzögerungszeiten die Tachykardie nicht beendet sein, so kann der gleiche Zyklus mit einer unterschiedlichen Zahl jeweiliger Stimulierungsimpulse wiederholt werden. Die Variationsmöglichkeit der Anordnung wird damit wesentlich erhöht. So kann nicht nur die Verzögerungszeit variiert werden, sondern auch die Anzahl der jeweils erzeugten Impulse und darüber hinaus auch noch der Abstand zwischen diesen Impulsen. Weiterhin kann auch festgelegt werden, in welcher Reihenfolge unterschiedliche Ablaufzyklen zur Anwendung kommen sollen.

Die unterschiedlichen Variationsmöglichkeiten lassen sich besonders einfach und vorteilhaft ausnutzen, wenn zumindest ein Teil der verschiedenen einstellbaren und/ oder vorgebbaren Grossen programmierbar ist. Eine besonders einfache Anordnung dafür besteht darin, dass bis auf das Eingangsteil und den Ausgangskreis alle Komponenten der Anordnung durch einen Mikroprozessor gebildet sind. In diesen können beispielsweise als Variable einprogrammiert werden,

1. die Tachykardiebedingungen,
2. die kürzeste Verzögerungszeit, üblicherweise wird diese knapp grösser als die Refraktärzeit bei Tachykardie gewählt,
3. die Anzahl der Stimulierungsimpulse, die synchron zu den Herzschlägen erzeugt werden sollen (beispielsweise zwei, drei oder mehr) mit einer eventuellen Steigerungsmöglichkeit,
4. der zeitliche Abstand zwischen den Stimulierungsimpulsen einer Gruppe.

Weiterhin sollte die Möglichkeit bestehen festzulegen ob die Anordnung automatisch beim Auftreten einer Tachykardie zu arbeiten beginnt oder ob die Anordnung aktiviert werden muss, beispielsweise von aussen durch Auflegen eines Magneten. Weiterhin kann auch festgelegt werden, wie lange die Anordnung den Versuch unternehmen soll, eine Tachykardie zu beenden, da Fälle auftreten können, in denen es nicht möglich ist, mit Hilfe oder nur mit Hilfe von Stimulierungsimpulsen eine Tachykardie zu beenden. Wird die Verordnung zusammen mit einem implantierten Herzschrittmacher verwendet, so ist es vorteilhaft, wenn auch noch die Basis-Inhibierungsfrequenz dieses Herzschrittmachers programmierbar ist. Zur Unterstützung des Herzens nach einer aufgetretenen Tachykardie ist es vorteilhaft, wenn nach deren Beendigung noch einige Stimulierungsimpulse mit normaler Herzfrequenz an das Herz abgegeben werden, um dieses wieder in den richtigen Rhythmus einzuschwingen.

Ohne den Rahmen der vorliegenden Erfindung zu verlassen, sind noch eine Vielzahl anderer, hier nicht im einzelnen erwähnter Kombinations- und Variationsmöglichkeiten gegeben.

Beispielsweise kann die Anordung in wie aus der europeischen Patentenmeldung Veröffentlichungsnr. 8505 bekannten Weise ganz oder teilweise in einer externen Einrichtung untergebracht sein, die mit einem implantierbaren Herzschrittmacher zusammenwirkt und diesen bei einer auftretenden Tachykardie ansteuert.

Anhand von zwei in vier Figuren dargestellten Ausführungsbeispielen wird im folgenden die Erfindung näher beschrieben und erläutert. Dabei zeigen

Fig. 1 ein Blockschaltbild eines ersten Ausführungsbeispieles der erfindungsgemässen Anordnung,

Fig. 2 in einer etwas detaillierteren Darstellung einen Teil der Anordnung nach Fig. 1,

Fig. 3 ein Blockschaltbild eines weiteren Ausführungsbeispieles und

Fig. 4 ein Impulsdiagramm zur Verdeutlichung der Funktionsweise der Anordnung.

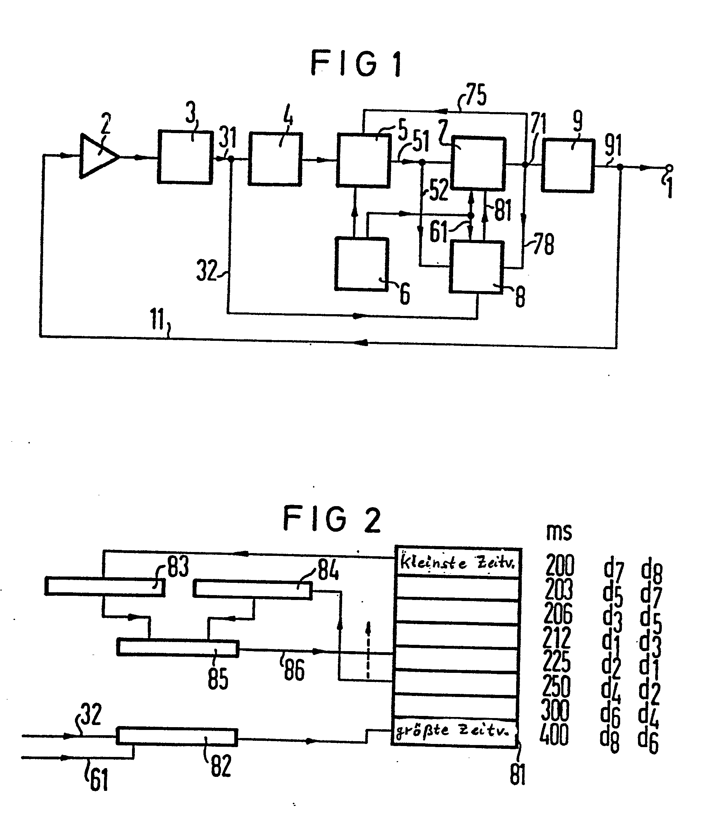

In Fig. 1 ist mit 1 ein Anschluss gekennzeichnet, an den eine zum Herzen führende Elektrode angeschlossen sein kann. Über diese Elektrode können sowohl Impulse dem Herzen zugeführt, als auch die Herzaktivität abgefühlt werden. Registrierte Herzschläge werden über eine Leitung 11 einem Eingangsverstärker 2 zugeführt. Dessen Ausgangssignal gelangt auf einen nur für Herzsignale ansprechenden Detektor 3. Die diesen Detektor passierenden Signale gelangen über eine Leitung 31 und ein Gatter 4 auf einen Rechner 5. Das Gatter 4 wird über einen Taktgeber 6 jeweils für eine bestimmte Zeitdauer geöffnet, beispielsweise für zwei Sekunden. Erreicht in dieser Zeit die Zahl der auf den Rechner gelangenden Impulse einen vorgebbaren Wert oder übersteigt diesen Wert, so liegt eine Tachykardie vor, und der Rechner erzeugt ein Ausgangssignal, das über eine Leitung 51 einen weiteren Rechner 7 startet.

Weiterhin ist ein Speicher 8 mit interner Recheneinheit vorgesehen, der in Fig. 2 näher beschrieben ist. Über eine Leitung 81 wird vom Speicher 8 ein bestimmter Wert für eine Verzögerungszeit in den Rechner 7 eingegeben. Weiterhin erhält der Rechner 7 vom Taktgeber 6 Taktimpulse mit einer Frequenz von beispielsweise einem Khz. Das Ausgangssignal des Rechners 7 ist über eine Leitung 71 auf einen Ausgangskreis 9 gegeben, der, angesteuert durch dieses Signal, eine vorgebbare Anzahl von Stimulierungsimpulsen über die Leitung 91 an den Anschluss 1 abgibt. Das Ausgangssignal des Rechners 7 ist weiterhin über eine Leitung 75 auf den Rechner 5 gegeben und dient dort als Rücksetzsignal. Weiterhin ist der Ausgang des Rechners 7 über eine Leitung 78 mit dem Speicher 8 verbunden und steuert dadurch eine Änderung des vom Speicher euf den Rechner 7 gegebenen Verzögerungszeitwertes. Der Speicher 8 wird weiterhin mit dem hochfrequenten Tektsignal beaufschlagt. Über eine Leitung 32 ist ausserdem das Ausgangssignal des Detektors 3 auf den Speicher 8 gegeben. Als weiteres Steuersignal wird über eine Leitung 52 das Ausgangssignal des Rechners 5, d.h. praktisch des Tachykardiedetektors, auf den Speicher 8 gegeben.

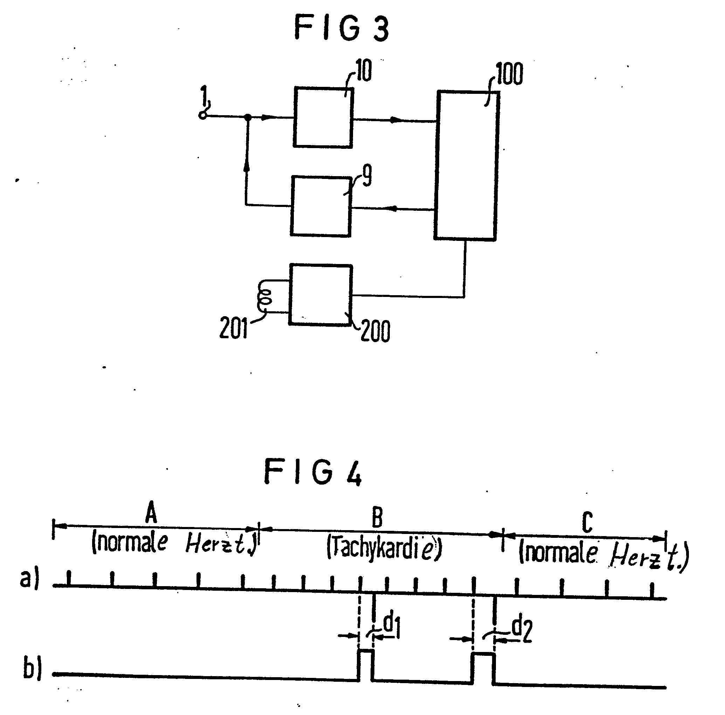

Die Funktionsweise dieser Anordnung wird im folgenden mit Hilfe der Impulsdiagramme gemäss der Fig. 4 erläutert:

Liegt eine normale Herztätigkeit vor, d.h. liegt die Frequenz der Herzschläge unter beispielsweise 150 Schlägen pro Minute, so erreicht der Rechner 5 während der Zeit, in der das Gatter 4 geöffnet ist, nicht den notwendigen Wert, um ein Ausgangssignal zu erzeugen. Die Anordnung zur Erzeugung von Stimulationsimpulsen wird damit nicht gestartet. Dies ist in Fig. 4 im oberen Teil als Abschnitt A dargestellt. Der mittlere Teil der Fig. 4 gibt die registrierten Herzsignale an. Im unteren Teil ist im Abschnitt A dargestellt, dass keine Tachykardie angezeigt wird und damit auch kein Stimulierungsimpuls erzeugt wird.

Im Abschnitt B ist anschliessend eine Tachykardie angenommen. Der Rechner 5 gibt somit ein Ausgangssignal an den Rechner 7 ab und startet diesen. Dieser rechnet bei spielsweise von dem aus dem Speicher 8 übernommenen Verzögerungszeitwert $d_1$ mit der Taktfrequenz rückwärts bis Null und gibt anschliessend ein Ausgangssignal ab, das den Ausgangskreis 9 zur Erzeugung eines Stimulierungsimpulses ansteuert. Im unteren Teil der Fig. 4 ist die Verzögerungszeit als Rechteckimpuls dargestellt. Am Ende der Verzögerungszeit wird ein erster Stimulierungsimpuls abgegeben, der im mittleren Teil der Fig. 4 als negativer Impuls dargestellt ist. Im vorliegenden Beispiel liegt dieser Stimulierungsimpuls nicht im richtigen Zeitabstand zum vorhergehenden Herzschlag, um die Tachykardie zu beenden. Der Rechner 5 startet daher nach dem erneuten Auftreten der vorgegebenen Zahl von Tachykardieherzschlägen, im Beispiel der Fig. 4 vier derartige Herzschläge, erneut die verzögerte Erzeugung eines Stimulierungsimpulses, jedoch mit einer verlängerten Verzögerungszeit $d_2$. Es ist nun angenommen, dass der nach dieser Verzögerungszeit abgegebene Stimulierungsimpuls die Tachykardie beendet. Im Abschnitt C der Fig. 4 ist das Herz wiederum zu seinem normalen Herzrhythmus zurückgekehrt.

In der Fig. 1 ist schematisch angedeutet, dass jedesmal, wenn der Rechner 7 den Ausgangskreis ansteuert, gleichzeitig der Rechner 5 auf seinen Ausgangspunkt zurückgesetzt wird und weiterhin ein Signal an den Speicher 8 abgegeben wird, wodurch ein anderer Verzögerungszeitwert in den Rechner 7 eingegeben wird. Wird nach dieser Verzögerungszeit die Tachykardie beendet, d.h. tritt am Ende einer vorgebbaren Zeit kein Ausgangssignal am Rechner 5 auf, so wird über die Leitung 52 der Speicher 8 praktisch in entgegengesetzter Richtung angesteuert, so dass der Rechner 7 wieder den alten Verzögerungszeitwert erhält. Damit wird praktisch erreicht, dass sich die Anordnung an den richtigen Verzögerungszeitwert zur Beendigung einer Tachykardie "erinnert". Beim erneuten Auftreten einer Tachykardie wird der erste abgegebene Stimulierungsimpuls wieder mit der gleichen Zeitverzögerung an das Herz abgegeben.

Fig. 2 zeigt in einer etwas ausführlicheren Darstellung eine mögliche Ausführungsform des Speichers 8. Wesentlicher Bestandteil dieses Speichers ist ein Register 80 mit im vorliegenden Beispiel acht Registerplätzen. In den obersten Speicherplatz wird beispielsweise die kleinste mögliche Zeitverzögerung fest eingegeben. Diese Zeitverzögerung entspricht praktisch der Refraktärzeit oder ist nur unwesentlich grösser als diese. In den untersten Registerplatz wird die grösste mögliche Zeitverzögérung eingegeben, die dem Abstand zweier Tachykardieherzsignale entspricht. Diese Zeit wird beispielsweise in einem Rechner 82 ermittelt, dem über eine Leitung 61 Taktimpulse vom Taktgeber 6 und über eine Leitung 32 Herzsignale zugeführt werden. Die zwischen zwei Herzsignalen anfallende Zahl von Taktimpulsen ist ein Mass für die zu messende Zeitdauer. Der kleinste Wert für die Verzögerungszeit wird in ein Register 83 eingegeben, der grösste zunächst in ein Register 84. Aus diesen beiden Werten wird in einer Stufe 85 die halbe Summe gebildet und über eine Leitung 86 auf den nächsten Registerplatz des Registers 81 übertragen. Dieser Wert wird dann als neuer Wert in das Register 84 eingelesen, wonach sich der gesamte Vorgang solange wiederholt, bis alle Plätze des Registers 81 belegt sind.

In einem Beispiel ist angenommen, dass der Abstand zwischen zwei Tachykardieherzschlägen 400 Millisekunden betragt und dass die Refraktärzeit des Herzens und gleichgesetzt damit die kürzeste Verzögerungszeit 200 Millisekunden beträgt. Damit ergeben sich die neben den Registerplätzen angegebenen Verzögerungszeiten.

In der Spalte daneben ist angegeben, in welcher Reihenfolge diese Verzögerungszeiten an den Rechner 7 gemäss dem Ausführungsbeispiel der Fig. 1 abgegeben werden, wenn das Intervall zwischen zwei Herzschlägen zentrifugal abgesucht werden soll. Begonnen wird mit dem Wert $d_1 = 212$ Millisekunden. Es folgt die zweite Verzögerungszeit $d_2$ mit 225 Millisekunden, danach die dritte $d_3$ mit 206 usw. Da im Beispiel gemäss der Fig. 4 bereits die zweite Verzögerungszeit $d_2$ zum erfolgreichen Beenden der Tachykardie führte, stellt dieser Wert die erste Verzögerungszeit beim erneuten Auftreten einer Tachykardie dar. Die dritte Spalte neben dem Register 81 zeigt die Reihenfolge der in einem derartigen Fall auftretenden unterschiedlichen Verzögerungszeiten.

In der folgenden Fig. 3 ist ein weiteres Ausführungsbeispiel in einem Blockschaltbild dargestellt. Entsprechend der Fig. 1 ist wiederum der Anschluss für die zum Herzen führende Elektrode mit 1 bezeichnet. Von diesem Anschluss gelangen die Signale über eine Leitung auf eine Stufe 10, die sowohl Verstärker als auch Detektor enthält. Von dort aus gelangen die Signale auf einen Mikroprozessor, der die unterschiedlichen Zeitbestimmungen sowie Rechenoperationen und Verzögerungszeiten ermittelt und festlegt. Der Ausgang dieses Mikroprozessors 100 steuert wiederum einen Ausgangskreis 9 zur Erzeugung der Stimulierungsimpulse.

Weiterhin ist in der Fig. 3 angedeutet, dass der Mikroprozessor 100 über eine Stufe 200 mit einer Empfangseinrichtung 201 programmiert werden kann. Eine ähnliche Programmiervorrichtung kann auch für die Anordnung gemäss der Fig. 1 vorgesehen sein.

Beide beispielhaft angegebenen Ausführungsformen können Teil eines implantierten Herzschrittmachers sein, der noch bei weiteren Herzrhythmusstörungen Stimulierungsimpulse an das Herz abgeben kann.

Der einfacheren Darstellungsmöglichkeit wegen ist in den Ausführungsbeispielen jeweils nur ein Stimulierungsimpuls nach der Verzögerungszeit angegeben. Ohne Einschränkung soll es selbstverständlich auch möglich sein, jeweils eine Gruppe von mehreren Stimulierungsimpulsen mit bestimmten festlegbaren Abständen zueinander an das Herz abzugeben.

**Patentansprüche**

1. Anordnung zum Beenden einer Tachykardie mit einem Eingangsteil, das nur von Herzsignalen beeinflussbar ist, einer Einrichtung (82) zum Messen der Zeit zwischen zwei Tachykardie-Herzsignalen (RR-Zeit) und einem Tachykardie-Detektor, der an das Eingangsteil angeschlossen ist und beim Auftreten einer Tachykardie ein Ausgangssignal liefert, das einem Verzögerungsglied mit variabler, einstellbarer Verzögerungszeit zugeführt ist, das eine Recheneinheit enthält, die aus dem Zeitwert zwischen zwei Tachykardie-Herzsignalen mindestens eine Verzögerungszeit ableitet, die nicht kürzer ist als ein vorgebbarer, einstellbarer Wert, und dessen Ausgangssignal synchron zu den Herzsignalen einen Ausgangskreis ansteuert, der dadurch mindesten einen Stimulierungsimpuls erzeugt, der über eine Elektrode dem Herzen zuführbar ist, dadurch gekennzeichnet, dass die Recheneinheit aus dem Zeitwert eine vorgebbare Anzahl unterschiedlicher Verzögerungszeiten ableitet, dass die Differenz benachbarter Verzögerungszeiten zu grösseren Zeiten hin immer grösser wird, und dass das Verzögerungsglied die so bestimmten Verzögerungszeiten in einer vorbestimmten Reihenfolge durchläuft.

2. Anordnung nach Anspruch 1, dadurch gekennzeichnet, dass die Verzögerungszeiten so gewählt sind, dass das aus der der RR-Zeit und der kürzesten Verzögerungszeit gebildete Zeitintervall durch mehrmaliges Halbieren des Restintervalls gegen die kürzeste Zeit hin in immer kleiner werdende Stufen unterteilt ist.

3. Anordnung nach Anspruch 1 oder 2, dadurch gekennzeichnet, dass ein Speicher zur Aufnahme der Verzögerungszeiten vorgesehen ist.

4. Anordnung nach Anspruch 3, dadurch gekennzeichnet, dass die zum Beenden einer Tachykardie führende Verzögerungszeit in den Speicher (8) eingebbar ist und beim erneuten Auftreten einer Tachykardie die erste eingestellte Verzögerungszeit darstellt.

5. Anordnung nach Anspruch 4, dadurch gekennzeichnet, dass die weiteren Verzögerungszeiten "zentrifugal" durchlaufen werden.

6. Anordnung nach einem der Ansprüche 1 bis 5, dadurch gekennzeichnet, dass der Ausgangskreis (9) jeweils mehrere Stimulationsimpulse erzeugt, wobei der Abstand dieser Impulse untereinander fest vorgebbar ist.

7. Anordnung nach einem der Ansprüche 1 bis 6, dadurch gekennzeichnet, dass nach vollständigem Durchlaufen eines Verzögerungszeit-Zyklus direkt ein weiterer Zyklus startet, wobei die Zahl der Stimulierungsimpulse pro Verzögerungszeit und/oder der Abstand dieser Impulse voneinander variierbar ist.

8. Anordnung nach einem der Ansprüche 1 bis 7, dadurch gekennzeichnet, dass zumindest ein Teil der verschiedenen einstellbaren und/oder vorgebbaren Grössen programmierbar ist.

9. Anordnung nach einem der Ansprüche 1 bis 8, dadurch gekennzeichnet, dass bis auf das Eingangsteil (10) und den Ausgangskreis (9) alle Komponenten der Anordnung durch einen Mikroprozessor

10. Anordnung nach einem der Ansprüche 1 bis 9, dadurch gekennzeichnet, dass diese ein Sperrglied aufweist und erst nach Aktivieren dieses Gliedes startet.

11. Anordnung nach einem der Ansprüche 1-10, dadurch gekennzeichnet, dass diese in einem implantierbaren Herzschrittmacher integriert ist.

12. Anordnung nach einem der Ansprüche 1-10, dadurch gekennzeichnet, dass diese als externe Einrichtung ausgebildet ist, die mit einem implantierbaren Herzschrittmacher zusammenwirkt und diesen bei einer auftretenden Tachykardie ansteuert.

## Claims

1. An arrangement for arrestig tachycardia, having an input component which can be influenced only by heart signals, timing device (82) which measures the time interval between two tachycardia heart signals (RR-Time), and a tachycardia detector connected to the input component to monitor the occurrence of tachycardia and supply am output signal to a delay component which has a variable, adjustable delay time and contains a calculating und which uses the time interval between two tachycardia heart signals to talculate at least one delay time which is not shorter than a predeterminable, adjustable value, and give an output signal in synchronism with the heart signals to drive an output circuit which thereby produces at least one stimulation pulse which can be fed to the heart via an electrode, characterised in that from the time interval the calculating unit derives a predeterminable number of different delay times, that the difference between adjacent delay times increases in accordance with longer times and that the delay component works in a specified sequence through the delay times determined in this way.

2. An arrangement as claimed in Claim 1, characterised in that the delay times are selected to be such that the time interval, formed from the difference between the RR-Time and the shortest delay time, is divided into ever smaller stages by repeatedly halving the residual interval in the direction of the shortest lime.

3. An arrangement as claimed in Claim 1 or 2, characterised in that a store is provided to accommodate the delay times.

4. An arrangement as claimed in Claim 3, characterised in that the delay time which leads to the arresting or tachycardia can be input into a store (8) to represent the first set delay time on the re-occurrence of tachycardia.

5. An arrangement as claimed in Claim 4, characterised in that the further delay times are worked through "centrifugally".

6. An arrangement as claimed in one of the Claims 1 to 5, characterised in that the output circuit (9) in each case generates a plurality of stimulation pulses where the interval between these pulses can be predetermined.

7. An arrangement as claimed in one or the claims 1 to 6, characterised in that when a delay time cycle has been completely worked through, a further cycle directly commences, where the number of stimulation pulses per delay time and/or the interval between these pulses is variable.

8. An arrangement as claimed in one of the Claims 1 to 7, characterised in that at least a part of the various, adjustable and/or predeterminable values is programmable.

9. An arrangement as claimed in one of the Claims 1 to 8, characterised in that all the components of the arrangement except for the input component (10) and the output circuit (9), are formed by a microprocessor (100).

10. An arrangement as claimed in one ofthe Claims 1 to 9, characterised in that the arrangement includes a blocking component and does not start until this component has been activated.

11. An arrangement as claimed in one of the Claims 1 to 10, characterised in that the arrangement is integrated in an implantable heart pacemaker.

12. An arrangement as claimed in one of the Claims 1 to 10, characterised in that the arrangement consists of an external device which cooperates with an implantable heart pacemaker and actuates the latter on the occurrence of tachycardia.

## Revendications

1. Dispositif pour arrêter une tachycardie, comportant une partie d'entrée, qui n'est influencée que par des signaux cardiaques, un dispositif (82) servant à mesurer la durée entre deux signaux cardiaques de tachycardie (durée RR) et un détecteur de tachycardie, qui est raccordé à la partie d'entrée et fournit, lors de l'apparition d'une tachycardie, un signal de sortie qui est envoyé à un circuit de retard produisant un temps de réglage variable et qui contient une unité de calcul qui dérive, à partir de la durée entre deux signaux cardiaques de tachycardie, au moins une durée de retard qui n'est pas inférieure à une valeur réglable pouvant être prédéterminée et dont le signal de sortie commande, en synchronisme avec les signaux cardiaques, un circuit de sortie qui produit de ce fait au moins une impulsion de stimulation qui peut être envoyée au coeur par l'intermédiaire d'une électrode, caractérisé par le fait que l'unité de calcul dérive, à partir de la durée, un nombre pouvant être prédéterminé de durées de retard différent, que la différence entre des durées de retard voisines augmente de plus en plus en direction des durées plus longues et que le circuit de retard produit les temps de retard ainsi déterminé, selon une succéssion prédéterminée.

2. Dispositif suivant la revendication 1, caractérisé par le fait que les durées de retard sont choisies de telle manière que l'intervalle de temps formé par la différence entre la durée RR et la durée de retard la plus brève est subdivisé, au moyen d'une division réitérée par deux de l'intervalle restant, en des échelons devenant de plus en plus petits en direction de la durée la plus brève.

3. Dispositif suivant la revendication 1 ou 2, caracterisé par le fait qu'il est prévu une mémoire pour l'enregistrement des durées de retard.

4. Dispositif suivant la revendication 3, caractérisé par le fait que la durée de retard, conduisant à l'arrêt d'une tachycardie, peut être mémorisée dans la mémoire (8) et représente la première durée de retard réglée lors de l'apparition d'une nouvelle tachycardie.

5. Dispositif suivant la revendication 4, caractérisé par le fait que les autres durées de retard sont explorées "de façon centrifuge".

6. Dispositif suivant l'une des revendications 1 à 5, caractérisé par le fait que le circuit de sortie (9) produit respectivement plusieurs impulsions de simulation, l'intervalle entre ces impulsions pouvant être prédéterminé de façon fixe.

7. Dispositif suivant l'une des revendications 1 à 6, caractérisé par le fait qu'un nouveau cycle commence directement à la fin de l'écoulement complet d'un cycle de retard, le nombre des impulsions de stimulation pour chaque durée de retard et/ou l'intervalle entre ces impulsions étant modifiable.

8. Dispositif suivant l'une des revendications 1 à 7, caractérisé par le fait qu'au moins une partie des différentes grandeurs réglables et/ou pouvant être prédéterminde est programmable.

9. Dispositif suivant l'une des revendications 1 à 8, caractérisé par le fait qu'hormis la partie d'entrée (10) et le circuit de sortie (9), tous les composants du dispositif sont formés par un microprocesseur.

10. Dispositif suivant l'une des revendications 1 à 9, caractérisé par le fait qu'il comporte un circuit de blocage et démarre uniquement après l'activation de ce circuit.

11. Dispositif suivant l'une des revendications 1 à 10, caractérisé par le fait qu'il est intégré dans un stimulateur cardiaque implanté.

12. Dispositif suivant l'une des revendications 1 à 10, caractérisé par le fait gu'il est réalisé sous la forme d'un agencement externe qui coagit avec un stimulateur cardiaque implanté et attaque ce dernier à l'apparition d'une tachycardie.

# FIG 1

# FIG 2

| ms | | |
|---|---|---|
| kleinste Zeitv. | 200 | d7 | d8 |
| | 203 | d5 | d7 |
| | 206 | d3 | d5 |
| | 212 | d1 | d3 |
| | 225 | d2 | d1 |
| | 250 | d4 | d2 |
| | 300 | d6 | d4 |
| größte Zeitv. | 400 | d8 | d6 |

# FIG 3

# FIG 4